# EUROPEAN PATENT APPLICATION

(11) **EP 1 369 110 A1**
(43) Date of publication of application: **10.12.2003**
(21) Application number: 02708372.4
(22) Date of filing: 06.03.2002
(51) Int. Cl.: A61K 9/51, B01J 13/00, B01J 13/12

(54) **PRODUCTION OF NANOPARTICLES FROM METHYL VINYL ETHER COPOLYMER AND MALEIC ANHYDRIDE FOR THE ADMINISTRATION OF HYDROPHILIC PHARMACEUTICALS, MORE PARTICULARLY OF PURIC AND PYRIMIDINIC BASES**

(30) Priority: 06.03.2001 ES 200100530
(71) Applicant: INSTITUTO CIENTIFICO Y TECNOLOGICO DE NAVARRA, S.A., 31008 Pamplona (ES)
(72) Inventor: ARBOS VILA, Pau, E-31008 Pamplona (ES); MERODIO DE LA QUINTANA, Marta, E-31008 Pamplona (ES); ARNEDO HERNANDEZ, Amaia, E-31008 Pamplona (ES); RECARTE FLAMARIQUE, Félix, E-31008 Pamplona (ES); RENEDO OMAECHEVERRIA, Maria, J., E-31008 Pamplona (ES); IRACHE GARRETA, Juan, M., E-31008 Pamplona (ES); ESPUELAS MILLAN, Maria, Socorro, E-31008 Pamplona (ES)
(74) Representative: Elzaburu Marquez, Alberto
(86) International application number: ES0200098
(87) International publication number: WO02069938

(57) **Abstract**

Manufacture of nanoparticles on the basis of methyl vinyl ether and maleic acid for the administration of pharmaceuticals of an hydrophilic nature, in particular analogs of puric and pyrimidinic bases.

The nanoparticles are obtained by desolvation with an hydroalcoholic phase of a methyl vinyl ether and maleic acid copolymer solution in acetone. The particles obtained are next treated with cross-linking agents (diamines or proteins) for the purpose of prolonging their useful life and are, possibly, incubated with a pharmaceutical which will be transported on the surface. The nanoparticles can carry the pharmaceutical likewise encapsulated which would then be added during the desolvation. In the case of the nanoparticle-ligand conjugates, the nanoparticles previously obtained and containing inside the pharmaceutical to be transported are incubated with the ligand or molecule which will contribute the property of specifically recognising a particular receptor of the organism.

These pharmaceutical forms have as objective to improve the transport of the pharmaceutical or biologically active molecule to its site of action and/or absorption. This property improves the specificity and effectiveness of said pharmaceuticals.

## Description

### Technical field of the invention

The present invention is within the scope of the procedures for the production of non-biological vectors, nanoparticles and nanoparticle-ligand conjugates, for the transport and administration of pharmaceuticals or active biological molecules of hydrophilic nature through the use of the methyl vinyl ether and maleic anhydride copolymer, poly(methyl vinyl ether-co-maleic anhydride), as priority element.

### State of the art

The behavior of a drug after its administration in the organism is determined by a combination of different processes: distribution and elimination when the drug is administered by intravenous route; absorption, distribution, and elimination when an extravascular route is used. Each one of these processes mainly depends on the physico-chemical properties of the drug substance which are determined by its chemical structure.

After administering the drug by conventional means, it is distributed throughout the organism according to its molecular structure, which determines its physicochemical properties (molecular mass, lipophilia, pKa, crystalline structure, solubility), biochemical properties (affinity with plasmatic or tissue proteins, affinity with the membrane or intracellular receptors, sensibility to the enzymes responsible for its biotransformation, etc.) and pharmacological properties (ability to clear biological membranes, intensity of the action, etc.).

Numerous studies have shown that the classical pharmaceutical forms (tablets, capsules, suppositories, injections, etc.) only permit the modulation of the intensity and/or speed of release of the biologically active molecule into the blood circulation (absorption), but they have no effect on the subsequent stages of *in viv*o behavior of the drug (distribution and elimination).

During the distribution stage, the drug spreads to a large number or to every anatomical area of the organism. This implies: (i) the administration of doses higher to those which would theoretically be needed to obtain the required effect or (ii) the repeated administration of the drug. The therapeutic effect is attained in spite of the loss of an important fraction of the drug dose in different tissues other than those where it should act, resulting in unwanted side effects. This nonspecific distribution of the drug, tolerable for those with high therapeutic indexes (quotient between the dose that provokes the toxic effects and the therapeutic dose), could become the limiting factor for the clinical use of certain biologically active molecules (example: the 5-fluorouridine).

So as to obtain a more rational and better adapted therapeutic use, one of the most promising possibilities is that which uses the concept of vectorization for the increase in the efficiency and specificity of action of the drugs. This concept consists in associating a drug or a biologically active molecule to an appropriate vector or carrier. Although there are different types of vectors, in this study only the characteristics and properties of the non-biological vectors (liposomes, nanoparticles and conjugates) will be studied. The use of these new pharmaceutical forms permits the association between the drug and a system whose mission is to carry the drug to its pharmacological target instead of remaining in a free state. In fact, the use of non-biological vectors permits the masking of the physicochemical properties of the drug and, logically, the characteristics of the carrier are what permit control over the distribution stage. In the best of cases, the vector-drug pair should be inactive and remain stable in the different biological media between the site of administration and the place of action.

The most important potentialities that these vectors or non-biological carriers provide are the following (Couvreur & Puisieux. Adv. Drug Del. Rev., 10 (1993) 141-162):
· protection against the chemical, enzymatic or immunological inactivation of the drug between the administration site and the place of action;
· improvement in the transport of the biologically active molecule to places of difficult access and its penetration into the cell (in the case of infections located in the intracellular territories which are inaccessible by simple diffusion);
· increase in the specificity of action by a selective, effective, and regular concentration of drug in the cellular and/or molecular target In this way, with smaller doses, the obtained therapeutic activity is, at least, identical and the side effects are milder;
· decrease in the toxicity for certain organs by means of modifying the tissular distribution of the biologically active molecule that is carried;
· in some cases, a lengthening of the time of residence of the drug in the organism (interesting for those molecules with high clearance and a low biological half-life) and, control of its release. All of this implies a decrease of the frequency of drug intake and indirectly, an increase in the observance of the treatment on the part of the patient.

Within the group of non-biological vectors, colloidal systems of solid particle type with a size smaller than a micrometer stand out; they are also called nanoparticles. These are subdivided into matrix nanospheres and vesicular nanocapsules (Orecchioni e Irache, Formes pharmaceutiques pour application locale. Lavoisier Tech & Doc., Paris, 1996, 441-457). The nanocapsules are vesicular systems formed by an internal cavity which is surrounded by a membrane or polymeric wall. The nanospheres are matrix forms, formed by a tridimensional polymeric net. In both cases, the molecules of the biologically active substance can be dissolved, entrapped or adhered to the macromolecular structure (in the nanospheres) or encapsulated by the polymeric membrane (in the nanocapsules). Also, the biologically active substance may remain adsorbed on the surface of the nanoparticles.

Depending on the used material, the nanospheres can be prepared from:
- reactions of monomer polymerization;
- macromolecules of natural origin;
- preformed synthetic polymers.

The nanoparticles produced from preformed polymers are generally obtained by two different procedures: (i) application of techniques based on the emulsion of an organic solution of the polymer (Vanderhoff et al, US PAT, (1979) 4 177 177) and (ii) by processes derived from the desolvation of the polymer (Fessi et al, FRENCH PAT, (1986) 2 608 988). On the contrary, nanoparticles of natural macromolecules are generally obtained by means of (i) gelification techniques (Rajaonari-vony et al, J. Pharm.Sci, 82 (1993) 912-917), (ii) emulsion (Vanderhoff et al, US PAT, (1979) 4 177 177) and (iii) controlled desolvation (Many et al, Pharm Acta Helv., 53 (1978) 79-82). Generally, a supplementary stage is needed to estabilize the aforesaid systems.

In the organism, the distribution of the nanoparticles generally depends on their physicochemical characteristics (mainly their size and their surface properties). By intravenous route, the submicronic vectors (for example, the nanoparticles) are captured by the endothelial reticular system (ERS), also called mononuclear phagocyte system, principally located in the liver, spleen, lungs and marrow. This property that nanoparticles possess converts them into very interesting pharmaceutical forms for the administration of drugs destined to the treatment of diseases located in the ERS or in the treatment of diseases in which the cells of ERS possess a function (infections, cancerous processes, etc.). On the other hand, administration of nanoparticles through the mucous membranes (oral, nasal, ocular, etc. routes) enables the development of adhesive interactions which increase the time of residence of the pharmaceutical form in contact with the site of absorption or action of the drug (Ponchel & Irache, Adv. Drug Del. Rev., 34 (1998) 191-219).

For the design of pharmaceutical forms (based on nanoparticles) capable of vectorizing cells or tissues that are different from those which the nanoparticles normally distribute, active vectors (also called third generation vectors or nanoparticle-ligand conjugates can be used) are used. These systems are obtained by means of the binding of molecules capable of interacting specifically with cellular or molecular receptors to the surface of the nanoparticles. Among these molecules or ligands, polyethylenglycoles ("stealth" vectors; Allen et al., Cancer Res., 52 (1992) 2431-2439), certain monoclonal antibodies (Weinstein et al, Pharm. Therap., 24 (1984) 207-233), lectins (Irache et al, Biomaterials, 15 (1994), carbohydrates (Maruyama et al, Biomaterials, 15 (1994) 1035-1042), amines (Roser & Kissel, Eur. J. Pharm. Biopharm., 39 (1993) 8-12), vitamins (Russell-Jones & Westwood, US PAT (1992) 07/956.003; WO 92/17167), etc. can be used.

Nowadays, the preparation of nanoparticle pharmaceutical forms containing hydrophilic drugs is limited by several aspects of their formulation. In general, hydrophilic substances or drugs do not show any affinity for matrix lipophylic systems (for example, nanospheres). Therefore, production yields are very low and, logically, can not be applied to drug administration. Analogs of puric and pirimidinic bases such as oligonucleotides, 5-fluouridine or ganciclovir are included in this category of hydrophilic drugs.

Poly(methyl vinyl ether-co-maleic anhydride) synthetic copolymers have a wide range of applications in the agricultural field (commercial name: Agrimer® VEMA, ISP) as adyuvants and estabilizers in veterinary solutions, in seed, granules and tablet coatings, etc. These copolymers are also used in chemical and pharmaceutical fields (commercial name: Gantrez® Copolymers, ISP), mainly due to their capacity to form coating films and their high bioadhesive capacity (Esposito er al., Biomaterials, 15 (1994) 177-182). They have also been described as thickeners, complexing agents and hydrophilic colloids. Likewise, their use in sustained release systems such as transdermic patches or ocular matrixes has been proposed (Finne et al., Int. J. Pharm., 78 (1992) 237-241).

Their main advantages are their relatively low toxicity (LD50= 8-9 g/Kg by oral route) and their easy production, regarding both quantity and price.

Poly(methyl vinyl ether-co-maleic anhydride) copolymers have also been used to prepare microparticle vectorial systems by a process of complex coacervation (Mortada et al., J. Microencas., 4 (1987) 11-21).

Poly(methyl vinyl ether-co-maleic anhydride) copolymer nanospheres are new matrix systems of vectors of submicronic size. These are able to retain the biologically active substance thanks to (i) solution or entrapping inside the macromolecular structure or matrix, (ii) covalent bonds result of the interaction of the drug and the polymer anhydride groups and (iii) processes of adsorption by means of weak bonds.

The extremely high reactivity of this copolymer due to its cyclic anhydride groups makes it perfect to retain hydrophilic substances in its structure. They are also very interesting systems for the fixation of ligands to the nanoparticle surface, without having to use highly toxic organic reactants (glutaraldehyde and carbodiimides) .

A variety of current drugs (for example, 5-fluouridine, ganciclovir) and biotechnological products (for example, antisense oligonucleotides) can be mentioned as hydrophilic active ingredients. All of them are analogs of puric and pirimidinic bases. Other kind of molecules such as proteins, peptides, lectins and, in general, all those substances which have alcohol groups and/or primary amines (for example: lectins) can also be fixed.

The majority of the vectorial systems that have been described in the literature and are used to administrate hydrophilic substances require laborious manufacturing processes. Thus, for example, quite complex bonds with toxic reactions (Rimoli et al., J. Controlled Rel., 58 (1999) 61-68), multiple emulsions (Jeffery et al., Pharm. Res., 10 (1993) 362-367) or working with very high drug concentrations in conditions not applicable to the industrial field have been proposed.

The conditions of applicability that allow to ascertain of the industrial interest of poly(methyl vinyl ether-co-maleic anhydride) nanoparticle vectors, could be the following:
1. The obtaining of homogeneous populations with submicronic particle size. This favors the distribution, improves the transport of active substances to its place of action and increases the penetration in the target cells.
2. The use in the chemical-pharmaceutical field of abundant preformed copolymers with a low expense, for the administration of drugs.
3. The obtaining of particles in which part of the drug will be entrapped and other part will be bound to the matrix by a covalent bond. This favors the bi-phasic release of the drug. The entrapped fraction enables a very quick initial release, while the bound fraction is released in a controlled way over a long period of time. This period will depend on particle degradability and on the breakage of the bounds between the polymer and the biologically active molecule.
4. The obtaining of particles in which the drug is bound by a covalent bond to the surface. Like in the previous case, the drug will be released in a controlled way and depending on the velocity of the breakage of the bonds between the groups located on the nanoparticle surface and the biologically active molecule.
5. The use of the aforementioned nanoparticles, as a base to produce third generation vectors or nanoparticle-ligand conjugates capable of carrying the active principle to certain cells or tissues by a specific recognition system.
6. Working conditions at room temperature that avoid the possible degradation of thermolabile active substances of hydrophilic nature, and of chemical and/or biotechnological origin.
7. The obtaining of particles capable of protection against premature inactivation of the encapsulated active principles.

### Description of the invention

Poly(methyl vinyl ether-co-maleic anhydride) nanoparticles are obtained by a desolvation method consisting in the addition of a polar solvent (miscible with the copolymer solution) to an organic solution of the copolymer. Subsequently, a second non-solvent liquid is added, in this case an hydroalcoholic solution. In order to do this, the copolymer is dissolved in a predetermined volume of acetone and then an ethanol solution is added. Then a similar volume of water is added to this solution. The particles are instantly formed in the medium, which takes on the appearance of a milky suspension. The organic solvents (ethanol and acetone) are then eliminated by evaporation under reduced pressure. The particles remain in a stable aqueous suspension.

The poly(methyl vinyl ether-co-maleic anhydride) copolymer is insoluble in water, but after some time it hydrolyzes to an acid form which is soluble in water. Although this hydration process starts on contacting with water, it lasts approximately 10 hours. This is the reason why a supplementary stage is required. This stage consists in the stabilization of the particles by the cross-linkage of the particles with the purpose of increasing their stability in aqueous media. This also allows the modulation and control of the release of the encapsulated drug.

As cross-linking agents, polyfunctional chemical compounds, including a number of polyamines and polyhydroxyl molecules, can be used. The main cross-linking agents used are human serum albumin (HSA) and 1,3-diaminopropane (DP). Other macromolecules, such as bovine serum albumin (BSA), povidone and gelatin, have also shown to be good stabilizing agents.

The next manufacturing step consists in purifying the resultant nanoparticles in order to eliminate all the remains of the process, mainly polymer and cross-linker residues. To do this, different techniques such as ultracentrifugation or tangential filtration may be used. Finally, the purified nanoparticles may be lyophilized or freeze-dried for their long term storage and preservation.

Depending on the aqueous solubility of the drug, two different procedures may be used to associate a drug to the nanoparticles.

The first one consists in binding the drug (hydrophilic or with a low aqueous solubility) to the polymer in an organic solution. In order to do this, poly(methyl vinyl ether-co-maleic anhydride) and the drug are dissolved in a given volume of acetone. This solution is incubated and stirred at a predetermined temperature and for a fixed time. Then a given volume of ethanol is added, followed by the addition of a similar volume of water. The particles are immediately formed in the medium, and this suspension takes on a milky appearance. The organic solvents (ethanol and acetone) are removed by means of evaporation under reduced pressure and the particles remain in a stable aqueous suspension. The particles are cross-linked with one of the aforementioned products (mainly HSA and DP) and purified by ultracentrifugation or tangential filtration. Finally, the particles can be lyophilized.

The second one is a process for the loading of molecules with a high polarity, in which the drug is bound to the surface of freshly prepared nanoparticles. In this case, the poly(methyl vinyl ether-co-maleic anhydride) copolymer is dissolved in a given volume of acetone. Then, a given amount of ethanol is added followed by the addition of a similar amount of water. The particles are immediately formed in the medium, and this suspension takes on a milky appearance. The organic solvents (ethanol and acetone) are removed by means of evaporation under reduced pressure, and the particles remain in a stable, aqueous medium.

The freshly prepared nanoparticles are then incubated at a fixed temperature for a predetermined time with an aqueous solution of the drug and the solution is incubated at a predetermined temperature and for a fixed time. Afterwards, particles are stabilized by cross-linkage using one of the aforementioned agents (mainly HSA and DP) and purified by ultracentrifugation or tangential filtration. Finally, nanoparticles can be lyophilized.

Finally, to prepare ligand-nanoparticle conjugates, the manufacturing process is quite simple and is based on the incubation between the nanoparticles and the ligand to be bound to the surface of the carriers. Then, a supplementary purifying step is needed to eliminate the unbound ligand remains. As in the previous cases, the obtained conjugates can be lyophilized.

The following examples illustrate the present invention, but in no way do they pretend to limit the possible uses of the invention. In these examples, three different pharmaceuticals, either derivatives from puric or pyrimidinic bases, have been used. These drugs are the anti-cancer agent 5-fluorouridine, the antiviral ganciclovir and the antisense oligonucleotide ISIS 2922.

5-fluorouracile (2,4-dioxo-5-fluropyrimidine) and, its derivative, 5-fluorouridine (2,4-dioxo-5-fluoropyrimidine riboside) are analogs of the pyrimidines, and their molecules contain a fluoride atom. These drugs are usually used in the treatment of colon cancer, either alone or in combination with other drugs. They are also used in the treatment of breast, cervix, endometrium, gastrointestinal, head and neck, liver, ovary, pancreas, prostrate and skin tumors. The 5-fluorouridine has an antitumoral activity which is 100-times more potent than 5-fluorouracile and other derivatives of this drug (Jampel et al. Arch. Ophtal., 108 (1990) 430-435). However, its clinical use is limited due to the severe side effects (leukopenia, thrombocytopenia and gastrointestinal toxicity) when administered in a traditionally pharmaceutical form (Brusa et al., II Farmaco, 52 (1997) 71-81). Administration of this drug in the form of nanoparticles would allow an increase of the therapeutic index of this drug.

Ganciclovir, (9-[(1,3-dihydroxy-2-propoxy)methyl]guanine, is a synthetic nucleoside analogue. This molecule exhibits antiviral activity against some herpes viruses (Martin et al., J. Med. Chem., 26 (1983) 759-761). Furthermore, it is the most frequently used antiviral drug in the treatment of infections caused by human cytomegalovirus (CMV) in immunocompromised patients, mainly in those with the acquired immunodeficiency syndrome (AIDS), congenital immunodeficiency or in individuals who have undergone organ transplantation. The principal clinical manifestation is retinitis, which if left untreated, results in irreversible blindness (Markham & Faulde, Drugs, 48 (1994) 455-460). The use of nanoparticles would permit the obtaining of pharmaceutical dosage forms showing sustained release properties and controlled drug release. Besides, and given its small size, they would minimally affect vision when administered by intravitreous route.

In addition, during the last few years, ganciclovir has been used as an adjuvant for gene therapy involving suicide genes. This new therapeutical technique consists of administrating retrovirus, adenovirus or herpes simplex incorporating the thymidine quinase (tk) gene, followed by the adminisration of ganciclovir to the patient. The viral vectors permit the incorporation of tk into the dividing cells and, after expression, the ganciclovir is metabolized by the expressed enzyme in its phosphate derivative. This derivative is then transformed into ganciclovir-diphosphate and finally in ganciclovir-triphosphate, which is toxic for the cell and induces its apoptosis. This is being successfully used for the treatment of pancreas tumors (Carrio et al., Gene Ther., 6 (1999) 547-553), prostate adenocarcinomas (Herman et al. , Hum. Gene Ther., 10 (1999) 1239-1249) and hepatocarcinomas (Qian et al., Hum. Gene. Ther., 10 (1997) 349-358).

Antisense oligonucleotides are new therapeutic agents which are able to regulate the genetic expression of living organisms. This technology consists in the use of synthetic fragments of DNA or RNA, called oligonucleotides, which allow the stoppage of the production of illnesses related to the synthesized proteins. Antisense compounds block the transmission of genetic information between the nucleus and the place where the protein is produced inside the cell. There is a variety of applications that include the treatment of certain types of cancer, inflammatory pathologies and viral illnesses.

ISIS 2922 is an antisense oligonucleotide with a sequence complementary to messenger RNA in the human citomegalovirus region IE1 (Anderson et al., Antimicrob. Agents Chemother., 40 (1996), 2004-2011). This oligonucleotide blocks the expression of a protein essential for viral replication, thereby inhibiting the replication of the virus (Leeds et al., Drug Metabol. Dispos., 25 (1997) 921-926) with an *in vitro* efficiency of about 30-times greater than traditional therapies. However, physico-chemical oligonucleotide characteristics (high mass, charged, hydrophilic molecules) do not allow its free access into the cell where they have to act. The use of nanoparticles permits an increase of the penetration capacity of those substances and a protection against exo- and endonucleases.

The size and zeta potential of the nanoparticles were determined in a Zetamaster apparatus (Malvern Instruments/Optilas, Spain). The drugs, 5-fluoroudine (provided by Sigma; Alcobendas, Spain) and ganciclovir (Cymevene®, Roche; Madrid, Spain), were analyzed by High Performance Liquid Chromatography (HPLC), using a Hewlett Packard series 1050 (Germany). The antisense oligonucleotide, ISIS 2922, was obtained from Pharmacia Biotech (Cambridge, United Kingdom) and analyzed by zone capillary electrophoresis. The *Sambucus nigra* lectin was obtained from Vector Laboratories (USA) and determined by gel permeability chromatography. The samples of nanoparticles were lyophilized using a Virtis Genesis 12EL apparatus (USA).

### Example 1: Preparation of nanoparticles from poly(methyl vinyl ether-co-maleic anhydride) copolumer

The process described below is valid for the preparation of colloidal pharmaceutical forms, nanoparticle type, which can be used for the administration of drugs or biologically active molecules. In addition, these nanoparticles can also be used for the film coating of macroscopic pharmaceutical forms, such as tablets, granules, granulates, and minigranules.

### 1.1. Cross-linkage with 1,3-diaminopropane (DP)

100 mg of poly(methyl vinyl ether-co-maleic anhydride) copolymer are dissolved in 5 mL organic solvent (acetone). Then, under stirring, 10 mL of a miscible organic solvent (ethanol) and 10 mL distilled water are added.

The resulting mixture is stirred for homogenization during 5 minutes and the suspension of nanoparticles is evaporated under reduced pressure for organic solvents elimination. The final volume is adjusted with water (or an aqueous solution) to 10 mL.

The next step is to cross-link the resulting nanoparticles with 1,3-diaminopropane (DP). In order to do this, a DP solution is prepared in water (1% v/v), and a predetermined amount of cross-linking agent is added to the suspension of nanoparticles for incubation for 5 minutes.

The suspension is then purified by ultracentrifugation (12 minutes at 10,000 rpm) or tangential filtration. The supernatants are eliminated and the residues are resuspended in water or in an aqueous solution of mannitol (5% w/v). Finally, the resulting suspension of nanoparticles is lyophilized maintaining all its properties.

The resulting formulation of the nanoparticles suspension before liophilization is:
.- Poly(methyl vinyl ether-co-maleic anhydride) 1.0 % w/v
.- Mannitol 5.0 % w/v
.- 1,3-diaminopropane c.s.
.- Purified water c.s.p. 10 mL

The mean size of the obtained nanoparticles is lower than 300 nm and the carriers display a negative superficial charge. Table 1 shows the influence of the amount of DP (used to cross-link the nanoparticles) on the size and zeta potential of the resulting nanoparticles. From these results, it is clear that an increase in the amount of cross-linking agent used to harden the nanoparticles increases the particle size and decreases negative zeta potential of these colloidal carriers. This last fact proves the reaction between the anhydride groups from the surface of the particles and the cross-linking agent.

**Table 1.**

| **Effect of the quantity of the cross-linking agent (DP) on the size and zeta potential of the resulting nanoparticles.** | | |
|---|---|---|
| **DP Quantity (mL)** | **Size (nm)** | **Zeta potential (mV)** |
| 0 | 206.7±1.13 | -51.7±2.45 |
| 2 | 239.8 ±5.16 | -37.8 +1.13 |
| 3 | 270.9 ±4.13 | -27.7+2.26 |
| 5 | 270.1 ±10.53 | -23.7±0.85 |
| 10 | 275.0±11.33 | -23.7±0.78 |

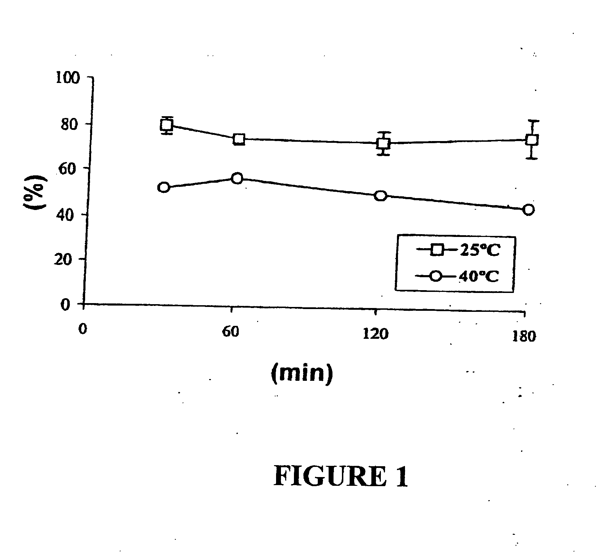

The yield of the nanoparticles manufacturing process was calculated as the quotient between the weight of the freeze-dried samples and the initial amount of polymer used to prepare the nanoparticles, either with mannitol or without cryoprotector. From a previously known amount of the polymer (100 mg) the amount that had been transformed in nanoparticles at the end of the process was determined by gravimetry. In any case, the yield of the manufacturing process (expressed in % with respect to the initial mass of polymer) was high; although higher efficiencies were obtained when the manufacturing process was carried out at room temperature rather than at 40°C (see Figure 1).

### 1.2. Cross-linkage with human serum albumin (HSA)

100 mg of poly(methyl vinyl ether-co-maleic anhydride) copolymer are dissolved in 5 mL of an organic solvent (acetone). Then, under magnetic stirring, 10 mL of a miscible organic solvent (ethanol) and 10 mL distilled water are added.

The resulting mixture is stirred for 5 minutes, and the suspension of nanoparticles is evaporated under reduced pressure for organic solvent elimination. The final volume is adjusted with water (or an aqueous solution) to 10 mL.

The next step is to harden the resulting nanoparticles with human serum albumin (HSA). In order to do this, a HSA solution is prepared in water (30 mg/mL), and a predetermined amount of cross-linking agent is added to the suspension of nanoparticles for incubation for 120 minutes.

The suspension is then purified by ultracentrifugation (12 minutes at 10,000 rpm) or tangential filtration. The supematants are eliminated and the residues resuspended in water or in an aqueous solution of mannitol (5% w/v). Finally, the resulting suspension of nanoparticles is freeze-dried maintaining all its properties.

The resulting formulation of the nanoparticles solution before liophilization is:
.- Poly(methyl vinyl ether-co-maleic anhydride) 1.0 % w/v
.- Mannitol 5.0 % w/v
.- Human serum albumin c.s.
.- Purified water c.s.p. 10 mL

The mean size of the obtained nanoparticles is lower than 300 nm and the carriers display a negative superficial charge. Table 2 shows the influence of the amount of HSA (used to cross-link the nanoparticles) on the size and zeta potential of the resulting nanoparticles. It was observed that an increase in the amount of cross-linking agent used to harden the nanoparticles slightly increases the size of the resulting nanoparticles and decreases their negative zeta potential. These results prove that the anhydride groups on the surface of the particles react with the cross-linking agent.

**Table 2.**

| **Effect of the quantity of the cross-linking agent HSA on the size and zeta potential of the resulting nanoparticles.** | | |
|---|---|---|
| **Amount HSA (mg/mg polymer)** | **Size (nm)** | **Zeta potential (mV)** |
| 0 | 206.7 ±1.13 | -51.7 ± 2.45 |
| 10 | 239.8 ±5.16 | -42.7 ±1.13 |
| 15 | 240.9 ± 15.05 | -42.7± 1.00 |

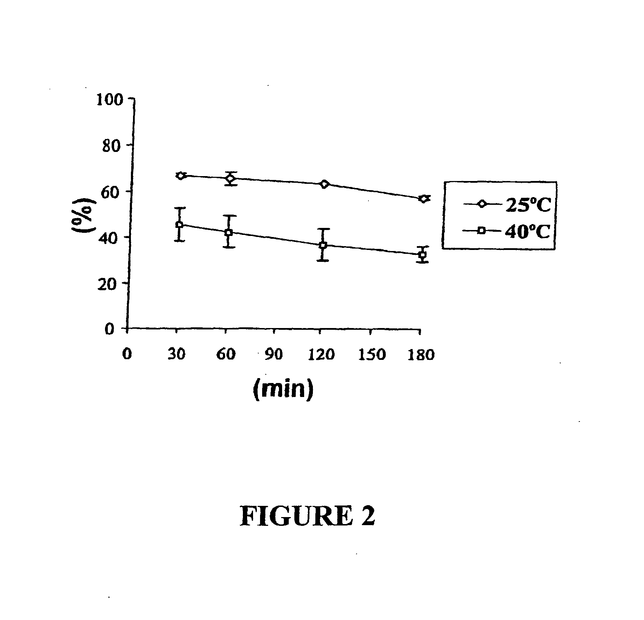

The yield of the nanoparticles manufacturing process was calculated as the quotient between the weight of the freeze-dried samples and the initial amount of polymer used to prepare the nanoparticles, either with mannitol or without cryoprotector. The amount of poly(methyl vinyl ether-co-maleic anhydride) copolymer that had been transformed in nanoparticles at the end of the process was determined by gravimetry from a previously known amount of the polymer (100 mg). In any case, the yield of the manufacturing process (expressed in % with respect to the initial mass of polymer) was high; although higher efficiencies were obtained when the manufacturing process was carried out at room temperature rather than at 40°C (see Figure 2).

### Example 2: Manufacturing process of nanoparticle conjugates based on the binding of lectin to the surface of poly(methyl vinyl ether-co-maleic anhydride) nanoparticles

The process described below permits the preparation of third generation pharmaceutical colloidal forms for the site specific delivery or targeting of drugs. These forms are formed by the binding of a ligand to the surface of copolymer nanoparticles. The ligand offers the possibility of driving the pharmaceutical dosage form to a specific site in the organism. In fact, the ligand should specifically recognize-certain receptors located on the surface or inside a certain type of cells. These forms are known as conjugates. In the next example, the model ligand used was the lectin of *Sambucus nigra* agglutinin (SNA).

First of all, 100 mg of poly(methyl vinyl ether-co-maleic anhydride) are dissolved in 5 mL organic solvent (acetone). Then, under magnetic stirring, 10 mL of a miscible organic solvent (ethanol) and 10 mL distilled water are added.

The resulting mixture is stirred for 5 minutes, and the suspension of nanoparticles is concentrated under reduced pressure for organic solvent elimination. The final volume is adjusted with water (or an aqueous solution) to 10 mL.

The next step is to harden the resulting nanoparticles with 1,3-diaminopropane (DP). In order to do this, a solution of DP is prepared in water (1% v/v), and a predetermined amount of cross-linking agent is added to the suspension of nanoparticles for incubation for 5 minutes. The suspension is then purified by ultracentrifugation (12 minutes at 10,000 rpm) or tangential filtration. The supernatants are eliminated and the residues resuspended in water.

Then the purified nanoparticles are incubated with the lectin. In order to do this, a variable amount of SNA is added to the nanoparticle suspension and this mixture is magnetically stirred at room temperature for 1 hour. The conjugates are centrifuged twice at 10,000 rpm for 12 minutes, in order to remove the unbound lectin (5% w/v).

Finally, the resulting suspension of conjugates is freeze-dried after the addition of sacarose or mannitol as cryoprotectors.

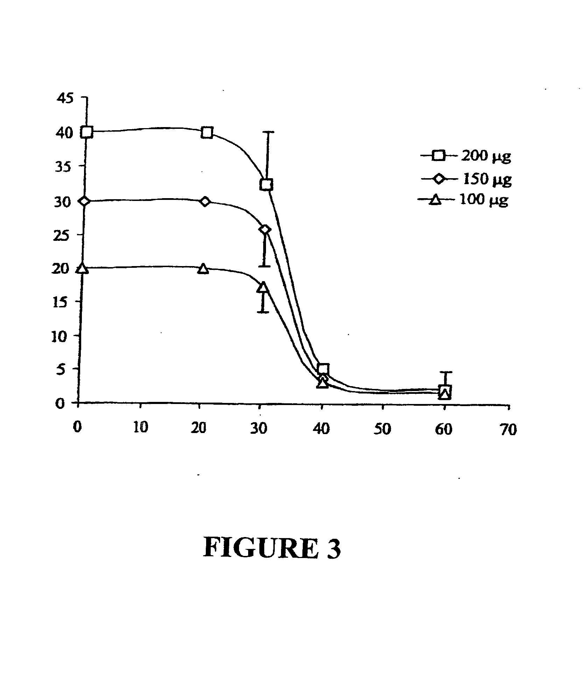

Figure 3 shows the influence of the initial amount of cross-linking agent on the lectin bound to the poly(methyl vinyl ether-co-maleic anhydride) nanoparticles. The lectin was determined by HPLC (High performance Liquid Chromatography). It was evident that as the amount of cross-linking agent increases, the amount of lectin being loaded by the nanoparticles decreases. Likewise, the amount of lectin bound to the nanoparticles is higher when the initial amount of lectin increases.

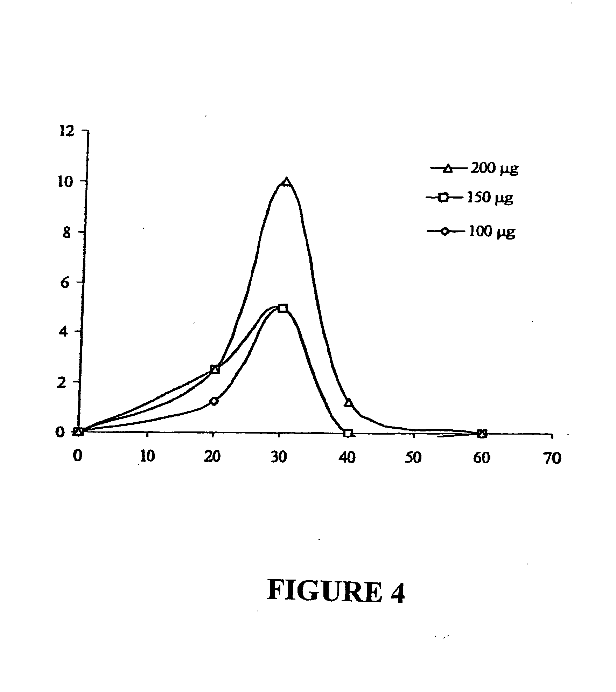

The quantity of active lectin bound to the nanoparticles was determined by means of an erythrocyte agglutination test. It was observed that by increasing the initial quantity of lectin, a greater amount of lectin bound to the nanoparticles remained active. When analyzing the influence of the amount of cross-linking agent, it was observed that there are certain ideal conditions that allow the optimal binding of lectins to the nanoparticle surfaces, around 30 µg/mg nanoparticle (Figure 4).

Similar results were obtained when using human serum albumin (HSA) or bovine serum albumin (BSA) as cross-linking agents.

### Example 3. Manufacturing process for the preparation of 5-fluorouridine-loading poly(methyl vinyl ether-co-maleic anhydride) nanoparticles

The process described below permits the preparation of colloidal pharmaceutical forms (i.e., nanoparticles and ligand-nanoparticle conjugates) carrying 5-fluorouridine or any other active molecule with a chemical structure similar to or derived from said drug.

### 3.1. Cross-linkage with 1,3-diaminopropane (DP)

100 mg of poly(methyl vinyl ether-co-maleic anhydride) are dissolved in 2 mL organic solvent (acetone). A predetermined amount of 5-fluorouridine is dissolved in 3 mL acetone, until a theoretical maximum of 10 mg/mL is reached.

The two aforementioned solutions are mixed and then incubated at room temperature for 2 hours. Then, under magnetic stirring, 10 mL of a miscible organic solvent (ethanol) and 10 mL distilled water are added to the acetone solution. The resulting mixture is stirred for 5 more minutes, and the suspension of nanoparticles is concentrated under reduced pressure for organic solvents elimination. The final volume is adjusted with water (or an aqueous solution) to 10 mL.

The next step is to harden the resulting nanoparticles with 1,3-diaminopropane (DP). In order to do this, a solution of DP is prepared in water (1% v/v), and a predetermined amount of cross-linking agent is added to the suspension of nanoparticles for incubation for 5 minutes.

The suspension is then purified by ultracentrifugation (12 minutes at 10,000 rpm) or tangential filtration. Optionally, the resulting nanoparticles (loaded with 5-fluorouridine) are incubated with the *Sambucus nigra* lectin in order to obtain the nanoparticle-lectin conjugates, as described in example 2. The supernatants are analyzed by HPLC. The quantity of entrapped drug in the nanoparticles is determined as the difference with respect to the initial quantity added. The residue or pellet is resuspended in water or in an aqueous solution of mannitol (5% w/v). Finally, the resulting suspension of nanoparticles is freeze-dried.

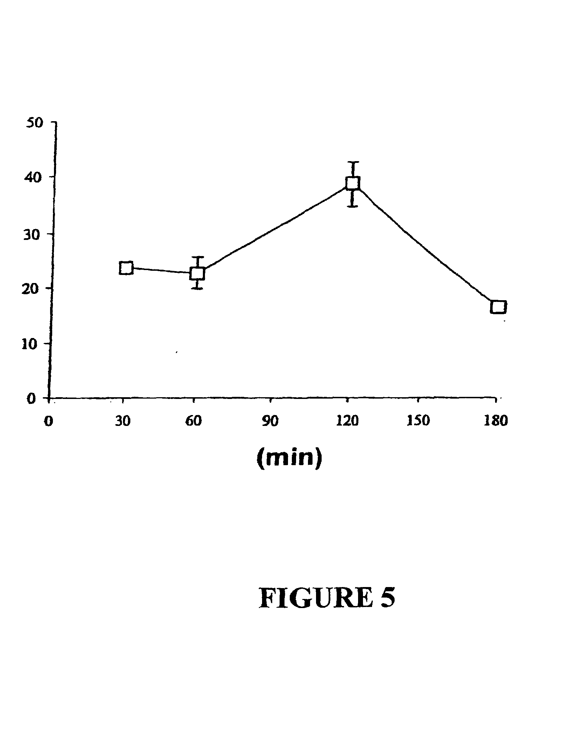

Figure 5 shows the influence of the incubation time between 5-fluorouridine and the poly(methyl vinyl ether-co-maleic anhydride) on the drug loading.

In the example of the SNA-nanoparticle conjugates containing 5-fluorouridine (experimental conditions: 200 µg initial lectin), the amount of encapsulated fluorouridine does not change and the amount of lectin bound to the surface of nanoparticles was calculated to be around 40 µg/mg polymer.

### 3.2. Cross-linkage with human serum albumin (HSA)

100 mg of poly(methyl vinyl ether-co-maleic anhydride) are dissolved in 2 mL organic solvent (acetone). A predetermined amount of 5-fluorouridine is dissolved in 3 mL acetone, until a theoretical maximum of 10 mg/mL is reached. The two aforementioned solutions are mixed and then incubated at room temperature for 2 hours.

Then, under magnetic stirring, 10 mL of a miscible organic solvent (ethanol) and 10 mL distilled water are added to the acetone solution. The resulting mixture is stirred for 5 more minutes, and the suspension of nanoparticles is concentrated under reduced pressure for organic solvent elimination. The final volume is adjusted with water (or an aqueous solution) to 10 mL.

The next step is to harden the resulting nanoparticles with human serum albumin (HSA). In order to do this, a solution of HSA is prepared in water (30 mg/mL), and a predetermined amount of cross-linking agent is added to the suspension of nanoparticles for incubation for 120 minutes.

The suspension is then purified by ultracentrifugation (12 minutes at 10,000 rpm) or tangential filtration. The supernatants are analyzed by HPLC. The quantity of entrapped drug in the nanoparticles is determined as the difference with respect to the initial quantity added. The residue or pellet is resuspended in water or in an aqueous solution of mannitol (5% w/v). Finally, the resulting suspension of nanoparticles is freeze-dried maintaining all its properties.

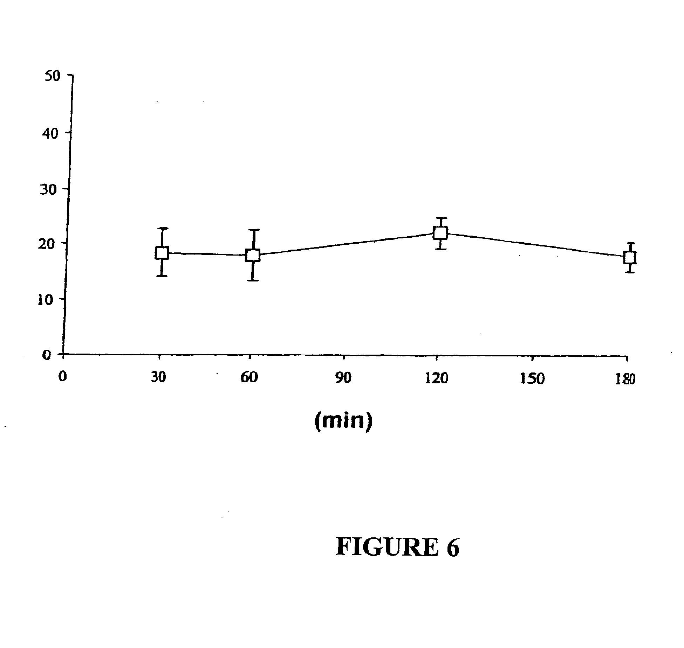

Figure 6 shows the influence of the incubation time between 5-fluorouridine and the copolymer on the drug loading of nanoparticles when the carriers where cross-linked with HSA.

### Example 4: Manufacturing process for the preparation of poly(methyl vinyl ether-co-maleic anhydride) nanoparticles coated with 5-fluorouridine

The process described below permits the preparation of colloidal pharmaceutical forms (i.e., nanoparticles and ligand-nanoparticle conjugates) containing 5-fluorouridine or any other active molecule with a chemical structure similar to or derived from said drug.

### 4.1. Cross-linkage with 1,3-diaminopropane (DP)

100 mg of poly(methyl vinyl ether-co-maleic anhydride) are dissolved in 5 mL organic solvent (acetone). A predetermined amount of 5-fluorouridine is dissolved in 2 mL water. Then, under magnetic stirring, 10 mL of a miscible organic solvent (ethanol) and 10 mL distilled water are added to the acetone solution of the copolymer.

The resulting mixture is stirred for 5 more minutes, and the suspension of nanoparticles is evaporated under reduced pressure for organic solvents elimination. The final volume is adjusted with water (or an aqueous solution) to 10 mL, and the suspension is then incubated with the 5-fluorouridine aqueous solution.

The next step is to harden the resulting nanoparticles with 1,3-diaminopropane (DP). In order to do this, a solution of DP in water (1% DP v/v water) is prepared and a predetermined amount of cross-linking agent is added to the suspension of nanoparticles for incubation for 5 minutes.

The suspension is then purified by ultracentrifugation (12 minutes at 10,000 rpm) or tangential filtration. The supernatants are analyzed by HPLC. The quantity of entrapped drug in the nanoparticles is determined as the difference with respect to the initial quantity added. The residue or pellet is resuspended in water or in an aqueous solution of mannitol (5% w/v). Finally, the resulting suspension of nanoparticles is freeze-dried maintaining all its properties.

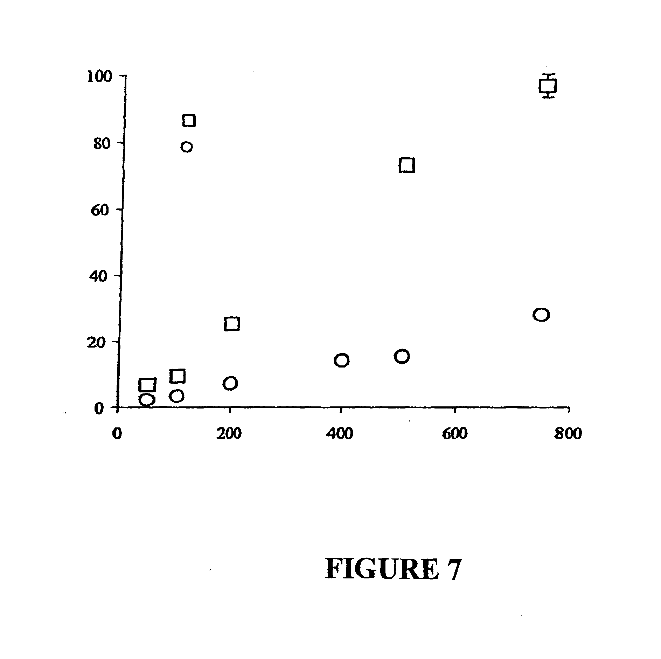

Figure 7 shows the influence of the initial amount of 5-fluorouridine (FU) that is adsorbed or interacts with the anhydride groups on the nanoparticle (NP) surface. It is clear that when the initial amount of the drug increases, the amount of FU carried by the nanoparticles also increases. Likewise, the higher the initial amount of FU loaded in the nanoparticles, the higher the amount that is released quickly. In the case of the administration of these colloidal pharmaceutical forms, the drug fraction that is released immediately would act as burst dose.

### 4.2. Cross-linkage with human serum albumin (HSA)

100 mg of poly(methyl vinyl ether-co-maleic anhydride) are dissolved in 5 mL organic solvent (acetone). A predetermined amount of 5-fluorouridine is dissolved in 2 mL water. Then, under magnetic stirring, 10 mL of a miscible organic solvent (ethanol) and 10 mL distilled water are added to the acetone solution of the copolymer.

The resulting mixture is stirred during 5 minutes more and the suspension of nanoparticles is concentrated under reduced pressure for organic solvents elimination. The final volume is adjusted with water (or an aqueous solution) to 10 mL and the suspension is, then, incubated with the 5-fluorouridine aqueous solution.

The next step is to harden the resulting nanoparticles with human serum albumin (HSA). In order to do this, a solution of HSA is prepared in water (30 mg/mL), and a predetermined amount of cross-linking agent is added to the suspension of nanoparticles for incubation for 120 minutes.

The suspension is then purified by ultracentrifugation (12 minutes at 10,000 rpm) or tangential filtration. The supernatants are analyzed by HPLC. The quantity of entrapped drug in the nanoparticles is determined as the difference with respect to the initial quantity added. The residue or pellet is resuspended in water or in an aqueous solution of mannitol (5% w/v). Finally, the resulting suspension of nanoparticles is freeze-dried maintaining all its properties.

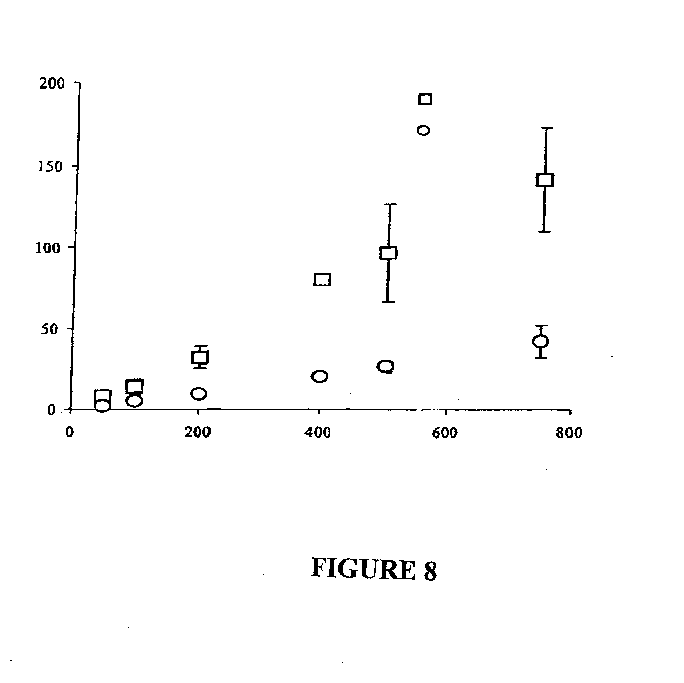

Figure 8 shows the influence of the initial concentration of 5-fluorouridine that is adsorbed and interacts with the reactive groups on the nanoparticle surface. It is clear that when the initial amount of drug increases, the amount of FU carried by nanoparticles cross-linked with HSA also increases. As expected, the immediately released fraction increased by increasing the amount of drug incubated with the freshly prepared nanoparticles. This burst effect would be acting as an initial dose.

### 4.3. In vitro release studies

For these studies, the nanoparticles were prepared following the conditions described in 4.1.

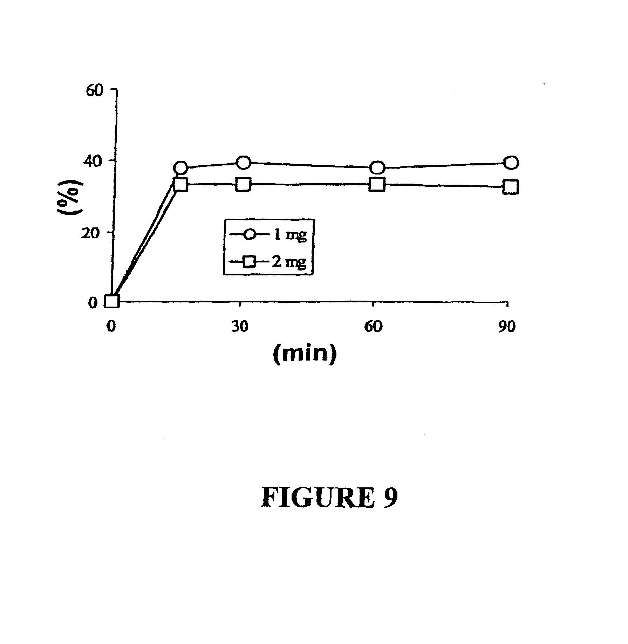

The *in vitro* release assay was carried out in a temperature-controlled bath, with constant stirring and a constant temperature of 37 ± 1° C. Figure 9 shows the results obtained for two different batches of nanoparticles prepared from 1 and 2 mg FU per mL suspension. It can be observed that a fraction of the drug (approximately 30-50% of the drug) is adsorbed, in a labile way, onto the surface of the nanoparticles and is immediately released. In addition, there is another fraction, which is strongly bound to the nanoparticles. This second fraction was not released during the time in which the release study was performed. However, it can be expected that *in vivo* conditions, the bond between the drug and the copolymer would break given its lability. This would permit the here described nanoparticles to act as a biphasic releasing drug. The great potential of this system is based on this property, together with their capacity to target certain organs and inflamed regions of the organism.

### Example 5: Manufacturing process for the preparation poly(methyl vinyl ether-co-maleic anhydride) nanoparticles coated with ganciclovir

The process described below permits the preparation of colloidal pharmaceutical forms (i.e., nanoparticles and ligand-nanoparticle conjugates) containing ganciclovir or any other active molecule with a chemical structure similar to or derived from said drug.

### 5.1. Cross-linkage with human serum albumin (HSA)

100 mg of poly(methyl vinyl ether-co-maleic anhydride) are dissolved in 5 mL organic solvent (acetone). A predetermined amount of ganciclovir is dissolved in water. Then, under magnetic stirring, 10 mL of a miscible organic solvent (ethanol) and 10 mL distilled water are added to the acetone solution of the copolymer.

The resulting mixture is stirred during 5 more minutes, and the suspension of nanoparticles is concentrated under reduced pressure for organic solvents elimination. The final volume is adjusted with water (or an aqueous solution) to 10 mL, and the suspension is then incubated with the ganciclovir aqueous solution.

The next step is to harden the resulting nanoparticles with human serum albumin (HSA). In order to do this, a solution of HSA is prepared in water (30 mg HSA/mL water) and a predetermined amount of cross-linking agent is added to the suspension of nanoparticles for incubation for 120 minutes.

The suspension is then purified by ultracentrifugation (12 minutes at 10,000 rpm) or tangential filtration. The supernatants are analyzed by HPLC. The quantity of entrapped drug in the nanoparticles is determined as the difference with respect to the initial quantity added. The residue or pellet is resuspended in water or in an aqueous solution of mannitol (5% w/v). Finally, the resulting suspension of nanoparticles is freeze-dried maintaining its properties.

This manufacturing process gives similar results when nanoparticles are cross-linked with 1,3-diaminopropane (DP) in the conditions indicated in the previous examples.

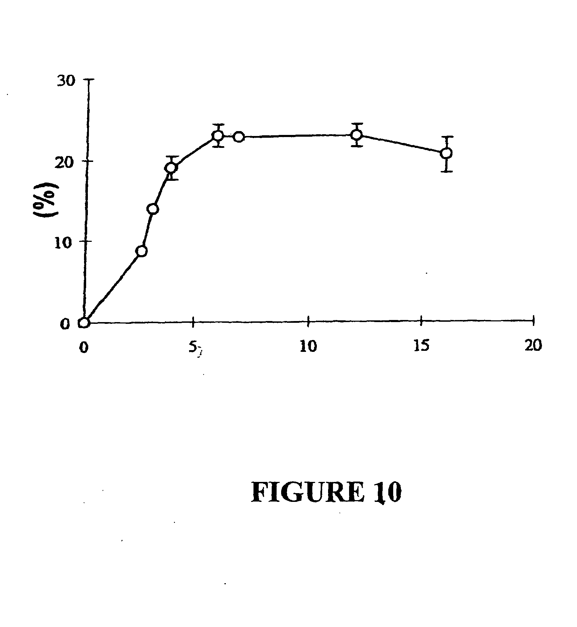

Figure 10 shows the influence of the bulk concentration of ganciclovir incubated with the nanoparticles in the percentage of drug bound to them. It can be observed that an initial ganciclovir amount greater, than 4-5 mg, does not have much effect on the percentage of entrapped drug.

### 5.2. In vitro release studies

These *in vitro* release studies were carried out with nanoparticulate batches prepared as described in 5.1. In order to do this, 100 µL of different formulation (with similar drug entrapped efficiency but increasing amounts of drug loaded) were dispersed in 1 mL phosphate buffer solution containing tripsin.

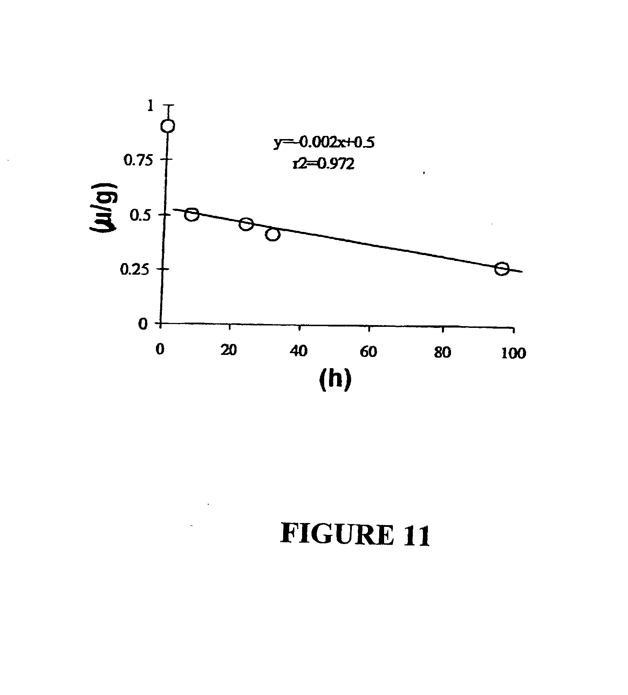

The *in vitro* release assay was carried out in a temperature-controlled bath, with constant stirring and a constant temperature of 37 ± 1° C taking samples at different predetermined times. A biphasic release profile was observed for all of the formulations tested. This profile was characterized by a first phase of rapid (almost immediate) release (also know as a "burst effect"), followed by a second slow and continuous release. During the first release stage (in the first eight hours), the percentages of drug released ranged between 40 and 50% of the loaded drug. The second part of the curve was adjusted to a zero-order kinetic (see Figure 11) that continued for more than 7 days. Figure 11 shows the ganciclovir release kinetic from the poly(methyl vinyl ether-co-maleic anhydride) nanoparticles, for a formulation containing 90 µg ganciclovir bound to the nanoparticles per mL suspension.

### Example 6: Manufacturing process for the preparation poly(methyl vinyl ether-co-maleic anhydride) nanoparticles coated with the antisense oligonucleotide ISIS 2922

The process described below permits the preparation of colloidal pharmaceutical forms (i.e., nanoparticles and ligand-nanoparticle conjugates) containing antisense oligonucleotides or any other active molecule of DNA or RNA. The example described below was carried out with the oligonucleotide ISIS 2922. ISIS 2922 is an oligonucleotide of 21 bases which possesses antiviral activity against cytomegalovirus.

### 6.1. Cross-linkage with 1.3 - diaminopropane (DP)

100 mg of poly(methyl vinyl ether-co-maleic anhydride) are dissolved in 5 mL organic solvent (acetone). A predetermined amount of ISIS 2922 is dissolved in water. Then, under magnetic stirring, 10 mL of a miscible organic solvent (ethanol) and 10 mL distilled water are added to the acetone solution of the copolymer.

The resulting mixture is stirred during 5 more minutes, and the suspension of nanoparticles is concentrated under reduced pressure for organic solvents elimination. The final volume is adjusted with water (or an aqueous solution) to 10 mL, and the suspension is then incubated with the ISIS 2922 aqueous solution for 20 minutes.

The next step is to harden the resulting nanoparticles with 1,3-diaminopropane (DP). For this purpose, a solution of DP is prepared in water (1% DP v/v water), and a predetermined amount of cross-linking agent is added to the suspension of nanoparticles for incubation for 5 minutes.

The suspension is then purified by ultracentrifugation (12 minutes at 10,000 rpm) or tangential filtration. The supematants are analyzed by capilar electrophoresis. The quantity of entrapped drug in the nanoparticles is determined as the difference with respect to the initial quantity added. The residue or pellet is resuspended in water or in an aqueous solution of mannitol (5% w/v). Finally, the resulting suspension of nanoparticles is freeze-dried maintaining its properties.

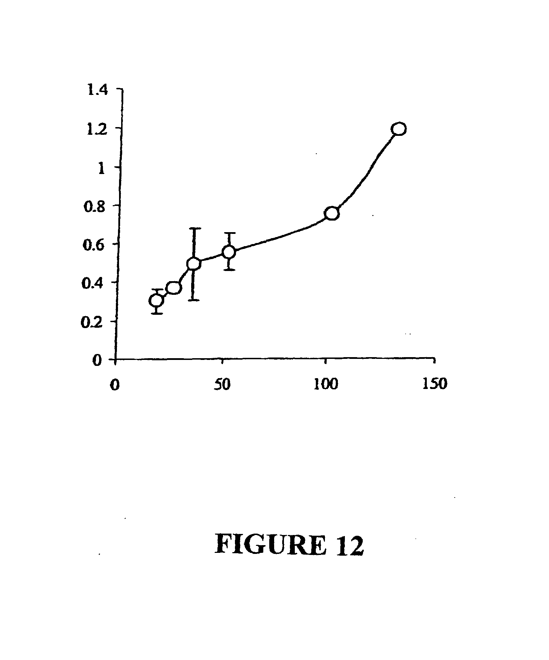

Figure 12 shows the influence of the initial amount of ISIS 2922 incubated with the nanoparticles on the amount of oligonucleotide loaded.

This procedure gives similar results when cross-linkage is carried out with (DP), in the conditions described in the previous examples.

### Description of the Figures

Figure 1. Influence of the incubation time (abscissas) and temperature on the yield of the manufacturing process (ordinates) of poly(methyl vinyl ether-co-maleic anhydride) nanoparticles cross-linked with DP.
Figure 2. Influence of the incubation time (abscissas) and temperature on the yield of the manufacturing process (ordinates) of poly(methyl vinyl ether-co-maleic anhydride) nanoparticles cross-linked with HSA.
Figure 3. Influence of the initial amount of the cross-linking agent (1,3-diaminopropane in µLsol/mg polymer; abscissas) on the amount of lectin bound (µg/mg polymer; ordinates) to the poly(methyl vinyl ether-co-maleic anhydride) nanoparticles.
Figure 4. Influence of the cross-linking agent (1,3-diaminopropane in µL·sol 1% v/v/mg polymer; abscissas) on the amount of active *Sambucus nigra* lectin (µg/mg polymer; ordinates) bound to the poly(methyl vinyl ether-co-maleic anhydride) nanoparticles.
Figure 5. Influence of the incubation time (abscissas) on the anti-cancer 5-fluorouridine loading in poly(methyl vinyl ether-co-maleic anhydride) nanoparticles cross-linked with DP (drug loading in µg FU/mg nanoparticles; ordinates). Experimental conditions: initially 10 mg drug.
Figure 6. Influence of the incubation time (abscissas) on the 5-fluorouridine loading in poly(methyl vinyl ether-co-maleic anhydride) nanoparticles cross-linked with HSA (Drug loading in µg FU/mg nanoparticles; ordinates). Experimental conditions: initially 10 mg drug.
Figure 7. Influence of the initial amount of 5-fluorouridine (FU) (µg/mg polymer; abscissas) on the drug loading in poly(methyl vinyl ether-co-maleic anhydride) nanoparticles cross-linked with 1,3-diaminopropane (µg FU/mg NP; ordinates). The squares represent the total drug loading and the circles represent the drug immediately released by dilution. Experimental conditions: Initially 10 mg drug.
Figure 8. Influence of the initial amount of 5-fluorouridine (µg FU/mg polymer; abscissas) on the drug loading in poly(methyl vinyl ether-co-maleic anhydride) nanoparticles cross-linked with HSA (µg FU/mg NP; ordinates). The squares represent the total drug loading and the circles represent the drug immediately released by dilution. Experimental conditions: Initially 10 mg of drug.
Figure 9. Study of the release (abscissas) of two formulations cross-linked with DP and prepared from two different concentrations of 5-fluouridine (FU 5 ordintaes).
Figure 10. Influence of the initial amount of ganciclovir (Bulk GCV in mg; abscissas) on the drug encapsulation efficiency (in %, ordinates) in poly(methyl vinyl ether-co-maleic anhydride) nanoparticles cross-linked with HSA.
Figure 11. *In vitro* release kinetic (hours in abscissas) in an aqueous solution of tripsin, for a formulation of poly(methyl vinyl ether-co-maleic anhydride) nanoparticles with ganciclovir (GCV - µg ordinates).
Figure 12. Influence of the initial amount of ISIS 2922 (µg in abscissas) on its loading in poly(methyl vinyl ether-co-maleic anhydride) nanoparticles (µg ISIS/mg NP; ordinates). Initial amount of nanoparticles (NP)= 100 mg.

## Claims

1. A process for the manufacture of nanoparticles and ligand-nanoparticle conjugates with site specific delivery or targeting properties, able to carry drugs or biologically active molecules on their surface or in their interior **characterized by** the desolvation of poly(methyl vinyl ether-co-maleic anhydride) copolymer, dissolved in an organic solvent, and subsequent cross-linkage reaction with polyfunctional chemical compounds, including polyamines and polyhydroxyls.

2. A process according to claim 1, that comprises:
a).- desolvation of the poly(methyl vinyl ether-co-maleic anhydride) polymer dissolved in a polar organic phase at a concentration between 0.01 and 10% w/v, which may optionally contain a drug or biologically active molecule, with a hydroalcoholic solution in an organic phase/hydroalcoholic solution ratio of 1/1 to 1/10;
b).- elimination of the organic solvents by conventional methods such as filtration, centrifugation or evaporation, including the use of vacuum, among others;
c).- stabilization of the resulting nanoparticles with cross-linking agents;
d).- optionally, incubation of the nanoparticles with either the drug or the biologically active molecule or, alternatively, the ligand with targeting properties;
e).- purification of either the obtained nanoparticles or conjugates by conventional techniques such as ultracentrifugation, centrifugation, tangential filtration, among others;
f).- optional freeze-drying of the obtained nanoparticles or conjugates.

3. A process according to claims 1 and 2, **characterized in that** nanoparticles or conjugates are produced in a way which permits encapsulation of the drug or biologically active molecule in their interior, that comprises:
i) the addition of said drug or biologically active molecule in step a) and,
ii) optionally, the addition of a second drug or a different biologically active molecule or, alternatively, a ligand with targeting properties in step d).

4. A process according to claims 1 and 2, **characterized in that** nanoparticles are produced with the drug or biologically active molecule on outer layer, that comprises:
i) the addition of the aforesaid drug or biologically active molecule in step d) and,
ii) optionally, the addition of another drug or biologically active molecule in step a).

5. A process according to claims 1 and 2, **characterized in that** unloaded nanoparticles are produced consisting of carrying out step a) and step d) without adding any drug or biologically active molecule or ligand.

6. A process according to any of claims 1 to 5, **characterized in that** step c) is carried out by using polyamine or polyhydroxyl type polyfunctional reagents, including among them proteins and polymeric macromolecules such as non-ionic surfactants or polyvinylpyrrolidone.

7. A process according to any one of claims 1 to 5, **characterized in that** step c) is carried out using the cross-linking agent 1,3-diaminopropane (DP) at a concentration ranging between 0 and 1 mg DP/mg poly(methyl vinyl ether-co-maleic anhydride).

8. A process according to any one of claims 1 to 5, **characterized in that** step c) is carried out using albumin, such as human serum albumin or bovine serum albumin, at a concentration ranging between 0 and 10 mg albumin/mg poly(methyl vinyl ether-co-maleic anhydride).

9. A process according to any one of claims 1 to 8, **characterized in that** step f) is carried out by adding mannitol or sacarose as a cryoprotector agent at a concentration ranging between 0.1 and 10% in weight.

10. A process according to any one of claims 1 to 4 and 6 to 9, **characterized in that** the ligands used in the production of the nanoparticle conjugates have the characteristics of being able to recognize specific structures, or cellular or tissular receptors located on the surface or inside specific cell types in the organism.

11. A process according to claim 10, **characterized in that** the ligands used are lectins, carbohydrates, monoclonal antibodies, vitamins, amino acids, lipids or molecules of a peptidic nature.

12. A process according to claim 11, **characterized in that** the ligand is the Sambucus nigra lectin which is bound at a concentration ranging between 1 and 100 mg lectin/mg nanoparticles.

13. A process according to claims 1 to 4 and 6 to 10, **characterized in that** the drugs or the biologically active molecules incorporated inside the nanoparticles and conjugates possess a hydrophilic character and are soluble in organic polar solvents.

14. A process according to claim 13, **characterized in that** which the drug incorporated is the anti-tumor drug 5-fluorouridine.

15. A process according to claim 14, **characterized in that** 5-fluorouridine is dissolved in acetone at a concentration ranging between 0.1 and 3.33 mg/mL and then added to the poly(methyl vinyl ether-co-maleic anhydride) acetone solution, in order to obtain a drug/polymer ratio ranging between 0.01 and 0.4 mg/mg.

16. A process according to any one of claims 1 to 4, 6 to 9, and 13 to 15, **characterized in that** the drug or biologically active substance is carried on the surface of the nanoparticles and in which step d) of incubation is produced in an aqueous solution.

17. A process according to claim 16, **characterized in that** the drugs or biologically active molecules incorporated into the surface of the nanoparticles are of hydrophilic character or are soluble in aqueous solutions.

18. A process according to claim 17, **characterized in that** the drugs or biologically active molecules are either analogs of puric or pyrimidinic bases, or they are compounds of protein nature such as peptides, proteins, glycoproteins, lipoproteins, or they are carbohydrates.

19. A process according to claim 18, **characterized in that** the drug incorporated in the surface of the nanoparticles is the anti-tumor agent 5-fluorouridine.

20. A process according to claim 19, **characterized in that** 5-fluorouridine is dissolved in water and, subsequently, added to the suspension of nanoparticles at a concentration ranging between 10 and 1000 µg drug/mg polymer, obtaining concentrations greater than 200 µg of drug bounded to the surface per mg nanoparticles.

21. A process according to claim 17, **characterized in that** the incorporated drug is the antiviral agent ganciclovir.

22. A process according to claim 21, **characterized in that** the ganciclovir is dissolved in water and then added to a suspension of nanoparticles, at a concentration ranging between 0.1 and 20 mg drug/mg polymer, obtaining entrapment efficiencies greater than 20% of the initially added ganciclovir.

23. A process according to claim 17, **characterized in that** the incorporated drug is an oligonucleotide.

24. A process according to claim 23, **characterized in that** the incorporated drug is an antisense oligonucleotide.

25. A process according to claim 24, **characterized in that** the incorporated drug is the antisense oligonucleotide ISIS 2922.

26. A process according to claim 25, **characterized in that** ISIS 2922 is dissolved in water and later added to the suspension of nanoparticles at a concentration ranging between 0.1 and 200 µg drug/mg polymer, producing nanoparticles whose superficially-bounded drug concentration is greater than 2 µg per mg nanoparticle.

27. Nanoparticles or conjugates obtainable by a manufacturing process according to any one of the claims 1 to 26, **characterized by** having an approximate average size of less than 500 nm.

28. Nanoparticles or conjugates according to claim 27, **characterized by** showing a biphasic release profile, with a first phase of immediate release of up to 60% of the loaded drug or biologically active molecule, followed by a second phase in which the drug or biologically active molecule is released slowly and in a sustained release manner.

29. Nanoparticles or conjugates **characterized in that** they have the following composition:
13-99% w/w poly(methyl vinyl ether-co-maleic anhydride),
0.001-15% w/w cross-linking agent,
optionally, 0.001-15% w/w drug or biologically active molecule,
optionally, 0.01-4.5% w/w ligand with specific targeting properties,
optionally, 70-82% w/w cryoprotector.

30. Use of the nanoparticles and conjugates according to any one of claims 27 to 29 for the administration of drugs or biologically active molecules.

31. Use of the nanoparticles and conjugates according of claims 27 to 29 in the administration of third generation colloidal pharmaceutical forms.

32. Use of the nanoparticles and conjugates of claims 27 to 29 for the pellicular coating of macroscopic pharmaceutical forms such as tablets, granules, granulates and pellets.

33. Use of the nanoparticles and conjugates of claims 27 to 29 loading 5-fluorouridine in the preparation of compositions that are useful in the treatment of certain diseases such as colon cancer, cancers of the gastrointestinal tract, breast cancer, cancers of the cervix and endometrium, as well as cancers of the head and neck, liver, ovary, pancreas, prostrate and skin.

34. Use of the nanoparticles and conjugates of claims 27 to 29 loading ganciclovir in the preparation of compositions which are useful for the treatment of infections induced by human cytomegalovirus.

35. Use of the nanoparticles and conjugates of claims 27 to 29, loading ganciclovir, as adjuvant, in the preparation of gene therapy compositions, which incorporate suicide genes and, in particular, the thymidine quinase gene.

36. Use of the nanoparticles and conjugates of claims 27 to 29 loading the antisense oligonucleotide ISIS 2922 in the preparation of compositions useful for the treatment of infections induced by human cytomegalovirus.
